(19) Europäisches Patentamt
European Patent Office
Office européen des brevets

(11) EP 4 678 283 A1

(12) **EUROPEAN PATENT APPLICATION**
published in accordance with Art. 153(4) EPC

(43) Date of publication:
**14.01.2026 Bulletin 2026/03**

(21) Application number: **24797327.4**

(22) Date of filing: **12.04.2024**

(51) International Patent Classification (IPC):
***B01J 23/02*** (2006.01) ***B01J 35/64*** (2024.01)
***B01J 35/61*** (2024.01) ***B01J 37/00*** (2006.01)
***C07C 37/11*** (2006.01) ***C07C 39/06*** (2006.01)

(52) Cooperative Patent Classification (CPC):
Y02P 20/52

(86) International application number:
**PCT/KR2024/004910**

(87) International publication number:
**WO 2024/225671 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.04.2023 KR 20230055405**

(71) Applicant: **Hanwha Solutions Corporation**
**Jung-gu**
**Seoul 04541 (KR)**

(72) Inventors:
- **LEE, Jong Kwon**
**Daejeon 34128 (KR)**
- **YUN, Seong Ho**
**Daejeon 34128 (KR)**
- **CHOI, Min Suk**
**Daejeon 34128 (KR)**
- **CHUN, Jeong Hwan**
**Daejeon 34128 (KR)**
- **JEON, Bongsik**
**Daejeon 34128 (KR)**

(74) Representative: **Berggren Oy**
**P.O. Box 16**
**Fabianinkatu 21**
**00101 Helsinki (FI)**

(54) **EXTRUSION MOLDED CATALYST FOR ORTHO-ALKYLATION REACTION AND PREPARATION METHOD THEREOF**

(57) The present invention relates to a technology for an extrusion-molded catalyst for ortho-alkylation reaction and its preparation method, and more specifically, to an extrusion-molded catalyst for ortho-alkylation reaction that can minimize the mass diffusion resistance of the catalyst in the ortho-alkylation reaction of a phenolic compound to obtain an ortho-alkylation reaction product with high selectivity and conversion rate, and a method for preparing the ortho-alkylation reaction product using the same.

【FIG. 1】

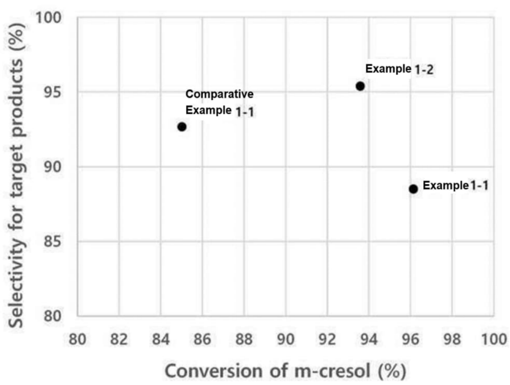


EP 4 678 283 A1

## Description

[TECHNICAL FIELD]

Cross-Reference to Related Application(s)

This application claims the benefit of priority based on Korean Patent Application No. 10-2023-0055405, filed on April 27, 2023, and all content disclosed in the aforementioned Korean patent application is incorporated herein by reference.

The present invention relates to a technology for an extrusion-molded catalyst for ortho-alkylation reaction and its preparation method, and more specifically, to an extrusion-molded catalyst for ortho-alkylation reaction that can minimize the mass diffusion resistance of the catalyst in the ortho-alkylation reaction of a phenolic compound to obtain an ortho-alkylation reaction product with high selectivity and conversion rate, and a preparation method thereof.

[BACKGROUND ART]

Alkylated hydroxyaromatic compounds are used for various purposes, and they are typically prepared by the gas-phase reaction of phenol and methanol. Furthermore, through additional alkylation reactions, compounds of various structures can be produced, and these are easily applicable to high-performance thermoplastic product lines.

These additional alkylation reactions have been typically performed in the presence of magnesium-based compounds, and various studies have been conducted to optimize the performance of magnesium-based catalysts.

In an alkylation reaction, a magnesium-based catalyst is required to have high activity, a long active life, and high selectivity for the desired reaction product. Most of the alkylation catalysts used in the past produced a large amount of para-alkylated products, and various studies have been conducted to obtain the more useful ortho-alkylated products with a high yield.

However, conventional techniques involve the combined use of cocatalyst compounds in addition to magnesium-based catalysts, modification of catalyst compositions, or alteration of reaction conditions. Accordingly, with such conventional techniques, it has been difficult to obtain ortho-alkylation products with the desired high selectivity and yield.

Accordingly, there is a need to develop an improved catalyst in terms of catalyst selectivity, catalytic activity, production yield, cost reduction, and total productivity in the ortho-alkylation reaction.

Meanwhile, for an alkylation reaction, it is possible to use a powder-form catalyst in small quantities at a laboratory level, but in order to mass-produce the catalyst and apply it to a commercial fixed-bed reactor, the catalyst must be appropriately molded to fit the reactor, considering the pressure drop during the reaction.

The methods commonly used for catalyst molding are extrusion molding and tablet molding. Tablet molding can produce a molded body with a precise shape, but the equipment is expensive and the preparing cost is high, which significantly reduces economic feasibility. On the other hand, extrusion molding is a commonly used molding method because the equipment is simple and the preparing cost is relatively low. However, when a catalyst is molded, it affects the internal/external mass diffusion resistance, which can cause a decrease in the activity of the prepared molded catalyst, so various studies are needed to minimize the activity decrease.

[DETAILED DESCRIPTION OF THE INVENTION]

[Technical Problem]

The present invention is to provide an extrusion-molded catalyst for ortho-alkylation reaction that can achieve an excellent conversion rate and yield in the production of a selective ortho-alkylation reaction product. Also, the present invention is to provide an extrusion-molded catalyst for ortho-alkylation reaction that can achieve uniform catalytic activity inside and outside the molded catalyst without a decrease in activity.

Furthermore, the present invention is to provide a preparation method for the catalyst.

[Technical Solution]

To solve the above problems, the present invention provides an extrusion-molded catalyst for ortho-alkylation reaction which comprises magnesium oxide, macropore having a diameter of 50 nm to 10,000 nm and mesopores having a diameter of 2 nm to 50 nm, and having a BET specific surface area of 45 $m^2/g$ to 180 $m^2/g$.

Furthermore, the present invention provides preparation method of the extrusion-molded catalyst for ortho-alkylation reaction.

Specifically, the present invention provides a preparation method of the extrusion-molded catalyst for ortho-alkylation reaction, comprising:

a first step of preparing a mixture of magnesium oxide having a bimodal pore structure and a BET specific surface area of 100 $m^2/g$ to 180 $m^2/g$, an organic binder resin, and a solvent; and
a second step of extrusion molding the mixture.

[ADVANTAGEOUS EFFECTS]

The extrusion-molded catalyst for ortho-alkylation reaction according to the present invention exhibits high catalytic activity, and when used to prepare an ortho-alkylation reaction product, it shows remarkably high selectivity and conversion rate.
Furthermore, the present invention provides an extrusion-molded catalyst for the ortho-alkylation reaction in which macropores and mesopores are appropriately formed and which has a suitable BET specific surface area, thereby minimizing the catalyst's mass-transfer resistance and enabling uniform, excellent catalytic activity both inside and outside the molded catalyst without any loss of activity.
The extrusion-molded catalyst for ortho-alkylation reaction according to the present invention is prepared by using a combination of magnesium oxide of specific physical properties, an organic binder resin, and a solvent, thereby exhibiting high catalytic activity on its own without the use of a separate co-catalyst or additional additives, and when used to prepare an ortho-alkylation reaction product, it exhibits remarkably high selectivity and conversion rate.

[BRIEF DESCRIPTION OF DRAWINGS]

FIG. 1 is a graph showing the conversion rate versus selectivity according to the type of catalyst in the molded catalysts for ortho-alkylation reaction of the Examples and Comparative Examples of the present invention.
FIG. 2 is a graph showing the conversion rate versus selectivity according to the type/content of the binder resin in the molded catalyst for ortho-alkylation reaction of the Examples of the present invention.
FIG. 3 is a graph showing the pore size versus pore volume according to the type of molded catalyst of the Examples and Comparative Examples of the present invention.

[DETAILED DESCRIPTION OF THE INVENTION]

Since the present invention can be subject to various modifications and can have various forms, specific embodiments will be illustrated and described in detail below. However, this is not intended to limit the present invention to the specific disclosed forms, and it should be understood to comprise all modifications, equivalents, or substitutes comprised in the spirit and technical scope of the present invention.
Furthermore, the terms used in this specification are used only to describe exemplary embodiments and are not intended to limit the present invention. Unless the context clearly indicates otherwise, singular expressions include plural expressions. In this specification, terms such as "include," "comprise," or "have" are intended to indicate the existence of an implemented feature, step, component, or a combination thereof, and should be understood as not precluding the possibility of the existence or addition of one or more other features, steps, components, or combinations thereof.
Furthermore, in the present invention, when a component is described as being formed "on" or "above" another component, it means that the component may be formed directly on the other component, or that an additional component may be formed between layers, on the object, or on the substrate.
Typically, a magnesium-based catalyst used in an alkylation reaction is required to have high activity, a long active life, and high selectivity for the desired reaction product. Most of the alkylation catalysts used in the past produced a large amount of para-alkylated products, and various studies have been conducted to obtain the more useful ortho-alkylated products with high selectivity.
Specifically, various techniques have recently been developed that combined use of cocatalyst compounds in addition to magnesium-based catalysts, modification of catalyst compositions, or alteration of reaction conditions. However, with these techniques, there was a problem of it being difficult to obtain the ortho-alkylated product with the desired high selectivity and conversion rate.
To solve these problems, the present inventors confirmed that for an extrusion-molded catalyst comprising a specific magnesium oxide, when the catalyst satisfies an appropriate pore distribution and BET specific surface area, it can achieve remarkably high catalytic activity in an alkylation reaction. Accordingly, the present inventors discovered that the ortho-alkylation reaction catalyst according to the present invention exhibits excellent catalytic activity as a single catalyst without additionally using a co-catalyst or adjusting reaction conditions, and when used to prepare an ortho-alkylation

reaction product, it can exhibit remarkably high selectivity and conversion rate, and thus completed the present invention.

Meanwhile, for an alkylation reaction, it is possible to use a powder-form catalyst in small quantities at a laboratory level, but in order to mass-produce the catalyst and apply it to a commercial fixed-bed reactor, the catalyst must be appropriately molded to fit the reactor, considering the pressure drop during the reaction. The methods commonly used for catalyst molding are extrusion molding and tablet molding. Tablet molding can produce a molded body with a precise shape, but the equipment is expensive and the preparing cost is high, which significantly reduces economic feasibility. On the other hand, extrusion molding is a commonly used molding method because the equipment is simple and the preparing cost is relatively low. However, when a catalyst is subjected to extrusion molding, it affects the internal/external mass diffusion resistance, which causes a problem of activity decrease in the prepared extrusion-molded catalyst.

Accordingly, to solve these problems, the present inventors identified the main factors affecting the catalytic activity of a molded catalyst when prepared by extrusion molding. Specifically, as described above, by using magnesium oxide having a specific pore structure, and at the same time, by appropriately forming macro-sized pores and meso-sized pores in the final catalyst to achieve a suitable BET specific surface area, the effect on internal/external mass diffusion resistance is minimized, and it was confirmed that uniform catalytic activity can be realized in the prepared extrusion-molded catalyst, thereby completing the present invention.

I. The extrusion-molded catalyst for ortho-alkylation reaction

An extrusion-molded catalyst for ortho-alkylation reaction according to an embodiment of the invention comprises magnesium oxide, macropores having a diameter of 50 nm to 10,000 nm and mesopores having a diameter of 2 nm to 50 nm, and satisfies a BET specific surface area of 45 $m^2/g$ to 180 $m^2/g$.

Generally, the catalytic activity of a catalyst is determined by the reaction conditions or the acid-base properties of the catalyst. In the alkylation reaction of a meta-alkyl substituted phenolic monomer, if the catalyst has basic properties, it adsorbs the reactant perpendicularly, which is favorable for ortho-C-alkylation (see figure (a) below). Accordingly, the ortho-alkylation reaction catalyst according to the present invention comprises a magnesium oxide (Magnesium oxide, MgO) component.

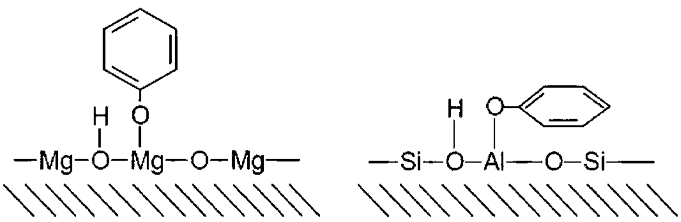

(a) Perpendicular Orientation Parallel Orientation

The catalyst is prepared using magnesium oxide having a bimodal pore structure, and the magnesium oxide used in the preparing step specifically has a bimodal form of medium-sized pores (meso-sized pores: mesopore), and accordingly, it facilitates the diffusion of reactants, allowing the reaction to be carried out effectively. Accordingly, it has excellent catalytic activity in the ortho-alkylation reaction and can exhibit high selectivity, conversion rate, and yield.

The extrusion-molded catalyst for ortho-alkylation reaction comprises macroporess having a diameter of 50 nm to 10,000 nm and mesopores having a diameter of 2 nm to 50 nm.

As such, macroporess and mesopores are appropriately formed to minimize the mass diffusion resistance of the catalyst, thereby enabling the realization of uniform and excellent catalytic activity inside and outside the molded catalyst without a decrease in activity.

The macropores of the extrusion-molded catalyst for ortho-alkylation reaction can be comprised in an amount of 1 to 10 vol% with respect to the total pore volume of the macropores and the mesopores, and preferably, can be comprised in an amount of 1 to 9 vol%, 2 to 10 vol%, 2 to 9 vol%, or 2 to 5 vol%. When comprised in this range, it is preferable because the mass diffusion resistance of the molded catalyst can be minimized.

The mesopores of the extrusion-molded catalyst for ortho-alkylation reaction can be comprised in an amount of 90 to 99 vol% with respect to the total pore volume of the macropores and the mesopores, and preferably, can be comprised in an amount of 90 to 98 vol%, 91 to 98 vol%, or 95 to 98 vol%. When comprised in this range, it is suitable for the molded catalyst to achieve excellent catalytic activity without a decrease in activity.

The method for measuring the pore size and pore volume distribution of the catalyst will be described in more detail in the experimental examples section below.

The extrusion-molded catalyst for ortho-alkylation reaction has a BET specific surface area of 45 $m^2/g$ to 180 $m^2/g$.

The catalyst, by having the appropriately formed macropores and mesopores and simultaneously exhibiting a BET specific surface area value in a specific range, can minimize the mass diffusion resistance of the catalyst, and can realize uniform and excellent catalytic activity inside and outside the molded catalyst without a decrease in activity.

Preferably, the BET specific surface area can be 45 $m^2/g$ to 150 $m^2/g$, 45 $m^2/g$ to 130 $m^2/g$, or 60 $m^2/g$ to 130 $m^2/g$, and when the specific surface area is within this range, it is preferable because the mass diffusion resistance of the molded catalyst can be minimized. The method for measuring the BET specific surface area of the catalyst will be described in more detail in the experimental examples section below.

Meanwhile, the pore distribution and BET specific surface area of the catalyst can be preferably realized by being prepared using a combination of magnesium oxide of specific physical properties, along with an organic binder resin and a solvent. This will be described in more detail in the preparation method section.

The extrusion-molded catalyst for the ortho-alkylation reaction may have a particle size of 0.5 mm to 6.0 mm, and within this range can exhibit uniform and excellent catalytic activity. More preferably, the catalyst particle size may be 1.0 mm to 3.0 mm or 1.2 mm to 2.0 mm. If the catalyst size deviates from the aforementioned range and becomes excessively small, a large pressure drop may occur during the alkylation reaction, which can be problematic. Conversely, if it deviates from the aforementioned range and becomes excessively large, there is a concern that the catalytic activity may decrease.

The size of the catalyst can be controlled according to the diameter of the extrusion die used in the final extrusion step in the preparing process.

Typically, the shape of an extrusion-molded catalyst is a cylindrical shape realized by an extrusion die, and the extrusion-molded catalyst according to the present invention can have a ratio of the diameter of the circular cross-section of the cylinder to the length of the cylinder of 1:1 ($\pm$0.1).

Therefore, the size of the extrusion-molded catalyst may refer to the diameter of the cylinder's circular cross-section and/or the length of the cylinder, and it is preferable that both the cross-sectional diameter and the cylinder length simultaneously fall within the aforementioned range.

The specific method for measuring the size of the catalyst will be described in more detail in the experimental examples section below.

II. A method for preparing the extrusion-molded catalyst for ortho-alkylation reaction

According to another embodiment of the invention, as a preparation method for the aforementioned extrusion-molded catalyst, a method for preparing the extrusion-molded catalyst for ortho-alkylation reaction comprising the following steps is provided.

Specifically, it comprises a first step of preparing a mixture of magnesium oxide having a bimodal pore structure and a BET specific surface area of 100 $m^2/g$ to 180 $m^2/g$, an organic binder resin, and a solvent; and a second step of extrusion molding the mixture.

Hereinafter, each step will be described in detail.

First step: Preparation of a mixture

First, a mixture is prepared by mixing magnesium oxide having a bimodal pore structure and a BET specific surface area of 100 $m^2/g$ to 180 $m^2/g$, an organic binder resin, and a solvent.

For the magnesium oxide, the description provided for the section of the extrusion-molded catalyst for ortho-alkylation reaction can be applied equally.

Preferably, the magnesium oxide has a bimodal pore structure, specifically a bimodal form in the range of medium-sized pores (pores with meso size: mesopores), and accordingly, it facilitates the diffusion of reactants, allowing the reaction to be carried out effectively. Accordingly, it has excellent catalytic activity in the ortho-alkylation reaction and can exhibit high selectivity, conversion rate, and yield.

Typically, the larger the BET specific surface area, the more catalyst active sites there are. However, in the case of a single-modal form, even if the BET specific surface area increases, the diffusion of reactants is not smooth, making it difficult to carry out the reaction to the desired extent.

Preferably, in the bimodal pore structure, the diameter of the first pore can be 2 nm to 10 nm, and the diameter of the second pore can be 10 nm to 50 nm, and more preferably, the diameter of the first pore can be 4 nm to 8 nm, and the diameter of the second pore can be 20 nm to 45 nm or 35 nm to 45 nm. Through the bimodal pore structure having a diameter in this range, it is possible to exhibit the desired excellent catalytic activity, improved selectivity, conversion rate, and yield.

The magnesium oxide has a BET specific surface area of 100 $m^2/g$ to 180 $m^2/g$, and has many reactive sites due to its relatively large specific surface area, resulting in excellent catalytic activity. At the same time, as described above, it has a bimodal pore structure, which facilitates the diffusion of reactants, allowing the reaction to be carried out effectively. Accordingly, it has excellent catalytic activity in the ortho-alkylation reaction and can exhibit improved selectivity,

conversion rate, and yield. If the BET specific surface area is less than 100 $m^2/g$, the number of reactive sites is significantly reduced, leading to a low reactant conversion rate, making it difficult to achieve the desired activity. Furthermore, if the BET specific surface area exceeds 180 $m^2/g$, the number of reactive sites increases, but the first pore is predominantly formed, which hinders diffusion of reactants and products and thus makes it difficult to achieve high activity.

Preferably, the BET specific surface area of the magnesium oxide can be 130 $m^2/g$ to 180 $m^2/g$, more preferably, 130 $m^2/g$ to 150 $m^2/g$, and within this range, it is preferable because excellent catalytic activity can be achieved without the aforementioned problems.

The organic binder resin is a component that facilitates catalyst preparation by uniformly binding the magnesium oxide during the preparation of the molded catalyst, and especially in the preparation of an extrusion-molded catalyst, it has an important effect on achieving uniformity of catalytic activity by forming appropriate pores inside the catalyst through calcination. If the magnesium oxide of specific physical properties described above is used alone, the ease of catalyst preparation may be reduced, and the formation of the desired pores may be somewhat difficult, but when used in combination, it is preferable because more excellent catalytic activity can be achieved uniformly.

Specific examples of the organic binder resin include methyl cellulose, carboxymethyl cellulose, ethylene glycol, polyethylene glycol, polyphenylene oxide, glycerin, and propylene glycol, and these may be used alone or in combination of two or more. More preferably, methyl cellulose, carboxymethyl cellulose, polyethylene glycol (PEG 20000 or PEG 400) can be used.

The organic binder resin can be comprised in an amount of 0.1 to 20 parts by weight, preferably 0.1 to 15 parts by weight, 0.1 to 10 parts by weight, or 1 to 5 parts by weight, based on 100 parts by weight of magnesium oxide. When used in this range, it is suitable for achieving the aforementioned effects.

The solvent is used so that the mixture can be kneaded uniformly, and it can facilitate the extrusion in the preparation of the extrusion molded body.

Specific examples of the solvent include water, alcohol, etc., and these may be used alone or in combination of two or more. More preferably, water can be used. The water may be high-purity water such as distilled water, ion-exchanged water, or deionized water (DIW). If the water contains impurities, the impurities may adhere to the catalyst and reduce the activity of the catalyst, so it is preferable to use deionized water or the like. In the case of alcohol, a C3 or higher primary alcohol can be preferably used.

The solvent can be comprised in an amount of 50 to 200 parts by weight, preferably 80 to 150 parts by weight or 100 to 120 parts by weight, based on 100 parts by weight of magnesium oxide. The mixture prepared at this ratio can preferably be in a paste form. In this case, the extrusion molding process can be easily carried out, which is preferable.

Meanwhile, according to an embodiment of the invention, optional additional additives may be used in the mixture; for example, lubricants may be used so that the final molded catalyst can be produced during the preparing process without unevenness or crushing. Specific examples of such lubricants include, but are not limited to, magnesium stearate, aluminum stearate, and graphite.

Furthermore, other additives used in the art may be used without particular limitation within a range that does not harm the desired physical properties.

Second step: Extrusion molding

Next, it comprises a second step of extrusion molding the mixture.

Tablet molding, which is a commonly used catalyst molding method, can produce a molded body with a precise shape, but the equipment is expensive and the preparing cost is high, which significantly reduces economic feasibility. On the other hand, extrusion molding is a commonly used molding method because the equipment is simple and the preparing cost is relatively low. However, when a catalyst is molded, it affects the internal/external mass diffusion resistance, which can cause a decrease in the activity of the prepared molded catalyst. In the case of the present invention, as described above, by using a combination of magnesium oxide of specific physical properties, an organic binder resin, and a solvent, it is possible to manufacture an extrusion-molded catalyst that has high activity and at the same time has uniformity inside and outside the catalyst without this problem.

The extrusion molding process can generally use a screw type (Screw extruder) and a piston type (Piston extruder), but preferably, it can be carried out using a single piston extrusion molding machine, and in this case, it is possible to manufacture an extrusion-molded catalyst with low moisture content and high compression ratio under high pressure. In this case, the extrusion process can be performed through a die of a specific diameter, but it is not limited thereto.

The average diameter of the extrusion die used can be appropriately adjusted according to the desired diameter range of the final molded catalyst.

Additional step(s): Drying and Calcination

In an embodiment of the invention, it may further comprise step(s) of drying and calcination after the extrusion molding.

The process conditions for the above-mentioned drying and calcination steps may be those conventionally employed in the art, without any particular limitation.

Specifically, the temperature at which the drying step is performed is not particularly limited; for example, the drying step may be carried out at 80°C to 120°C, and preferably at 90°C to 110°C.

The time for carrying out the drying step is not particularly limited, but for example, it can be carried out for 1 hour to 13 hours, and preferably for 3 hours to 8 hours, or 4 hours to 6 hours.

The temperature for carrying out the calcination step is not particularly limited, but for example, it can be carried out at 300°C to 600°C, and preferably at 400°C to 550°C.

The duration of the calcination step is not particularly limited, but it is carried out for a sufficient time to remove the organic binder resin and solvent from the mixture and thereby form the desired pores in the catalyst. Preferably, it is carried out for 1 to 9 hours, and more preferably for 3 to 6 hours.

The catalyst prepared according to the aforementioned method for preparing the extrusion-molded catalyst for the ortho-alkylation reaction, by using a combination of magnesium oxide with specific physical properties, an organic binder resin, and a solvent, can realize uniform and excellent catalytic activity both inside and outside the molded catalyst without any loss of activity.

III. ortho-alkylation reaction composition

According to an embodiment of the invention, an ortho-alkylation reaction composition comprises the aforementioned extrusion-molded catalyst for ortho-alkylation reaction and a monomer composition comprising a meta-alkyl substituted phenolic monomer.

The extrusion-molded catalyst for ortho-alkylation reaction comprises magnesium oxide, macropores having a diameter of 50 nm to 10,000 nm and mesopores having a diameter of 2 nm to 50 nm, and satisfies a BET specific surface area of 45 $m^2/g$ to 180 $m^2/g$. For this, all the aforementioned contents can be applied equally.

Specifically, the aforementioned macropores (macro pore) and mesopores (meso pore) are appropriately formed to minimize the mass diffusion resistance of the catalyst, thereby enabling the realization of uniform and excellent catalytic activity inside and outside the molded catalyst without a decrease in activity.

Furthermore, the magnesium oxide contained in the catalyst has a bimodal pore structure, specifically a bimodal form of medium-sized pores (mesopores), and accordingly, it facilitates the diffusion of reactants, allowing the reaction to be carried out effectively. In addition, by simultaneously satisfying the aforementioned specific range of BET specific surface area, it can exhibit excellent catalytic activity and high selectivity and conversion rate in the ortho-alkylation reaction.

In the monomer composition, the meta-alkyl substituted phenolic monomer can be m-cresol.

The monomer composition, in addition to the meta-alkyl substituted phenolic monomer, further comprises an alkanol and distilled water (DI Water), and an alkyl group can be introduced through the reaction with the alkanol, and by using the distilled water together, the decomposition reaction of the alkanol can be suppressed, which is preferable. The alkanol can preferably be methanol.

The monomer composition can preferably be comprised of phenolic monomer : alkanol : distilled water in a ratio of 1 : 3 to 10 : 1 to 5 parts by weight, and more preferably, can be comprised in a ratio of 1 : 4 to 6 : 1 to 3 parts by weight. At this time, if the content of alkanol is comprised in a small amount outside the above range, the conversion rate decreases due to a small amount of alkylating agent, and if the distilled water is comprised in a small amount outside the above range, the effect of suppressing the decomposition reaction of alkanol is reduced. If alkanol or distilled water is comprised in an excessive amount outside the above range, it will competitively adsorb with the phenolic monomer on the catalyst active sites, which may cause a problem of reduced reactivity.

When present within the above content range, it is preferable because the ortho-alkylation reaction product can be prepared with the desired selectivity and conversion rate.

IV. A preparation method of an ortho-alkylation reaction product

According to an embodiment of the invention, a preparation method of an ortho-alkylation reaction product comprises an alkylation reaction of a monomer composition comprising a meta-alkyl substituted phenolic monomer in the presence of the aforementioned extrusion-molded catalyst for ortho-alkylation reaction. Specifically, the preparation method of an ortho-alkylation reaction product can be carried out using an ortho-alkylation reaction composition comprising the aforementioned extrusion-molded catalyst for ortho-alkylation reaction. Regarding the catalyst and

reaction composition, all the aforementioned contents can be applied equally.

The extrusion-molded catalyst for ortho-alkylation reaction comprises magnesium oxide, comprises macropores having a diameter of 50 nm to 10,000 nm and mesopores having a diameter of 2 nm to 50 nm, and satisfies a BET specific surface area of 45 $m^2/g$ to 180 $m^2/g$. For this, all the aforementioned contents can be applied equally.

Specifically, by including the above-described extrusion-molded catalyst, uniform and excellent catalytic activity can be achieved both inside and on the outside of the molded catalyst without any decrease in catalytic activity.

In the preparation method of the ortho-alkylation reaction product, for the monomer composition, all the aforementioned contents can be applied equally, and specifically, the meta-alkyl substituted phenolic monomer in the monomer composition can be m-cresol. The preparation method of the ortho-alkylation reaction product, by using a reaction composition comprising the aforementioned extrusion-molded catalyst, can exhibit high selectivity and conversion rate of the desired ortho-alkylation reaction product.

The ortho-alkylation reaction product may be, for example, one or more selected from the group consisting of 2,5-dimethylphenol, 2,3-dimethylphenol, 2,3,6-trimethylphenol, 3-methylanisole, 3,4-dimethylphenol, and tetramethylphenol, and for example, it can be prepared by selectively substituting an alkyl group at the ortho position in m-cresol through a multi-step reaction as shown below.

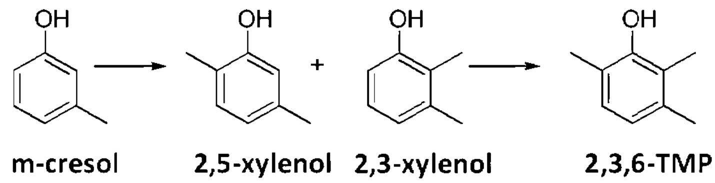

The alkylation reaction can be preferably carried out at 350°C to 550°C, and more preferably at 350°C to 550°C. At this time, if the temperature is below 350°C, the alkylation reaction may not be sufficiently activated, and if it exceeds 550°C, a large amount of by-products due to over-reaction will be produced.

The alkylation reaction can be carried out under inert conditions, for example, in the presence of an inert carrier gas. As the inert carrier gas, nitrogen, helium, neon, argon, etc., can be used, and preferably, nitrogen can be used.

The nitrogen flow rate is not particularly limited, but is preferably 5 to 70 cc/min, 10 to 50 cc/min, or 15 to 40 cc/min, and when the reaction is carried out under these conditions, the ortho-alkylation product can be produced with the desired selectivity and conversion.

Meanwhile, in the alkylation reaction, before the injection of the monomer composition, a process of raising and maintaining the inside of the reactor to the activation temperature for catalyst activation can be performed, and the temperature range may be the same as the reaction temperature. The time for catalyst activation is not particularly limited, but it can be about 30 minutes to 1 hour.

The alkylation reaction can be preferably carried out using a continuous flow gas-phase reaction apparatus, and in this case, the monomer composition can be injected into the continuous flow gas-phase reaction apparatus at an LHSV (liquid hourly space velocity) of 0.5 hr-1 to 2.0 hr-1. At this time, if it is outside the space velocity range, the reactant may not be sufficiently activated at the catalyst active site, making it difficult to obtain the product smoothly, and also, the activity or selectivity of the catalyst may be lowered, which may reduce the amount of product.

It is preferable to carry out the reaction under the above conditions, because the ortho-alkylation reaction product can be prepared with the desired selectivity and conversion rate

Hereinafter, the operation and effects of the invention will be described in more detail through specific embodiments. However, these embodiments are provided merely as examples of the invention and shall not be construed to limit the scope of the invention or its patent rights.

[Example 1 and Comparative Example 1]

Example 1-1. Preparation of Extrudate catalyst (preparation of extrusion-molded catalyst)

50 g of magnesium oxide (MgO powder, Crystallite size 9 nm, BET surface area 144.4 $m^2/g$, Total pore volume 0.72 $cm^3/g$, Pore size 6, 40 nm), 1 g of organic binder resin (methyl cellulose), and 60 g of distilled water were placed in a mixing bowl and mixed well to prepare a paste. When the kneading was completed, it was extruded through a 2 mm diameter die using a single piston extruder catalyst molding machine. The noodle-like shape extruded from the single piston extruder was spread evenly on a tray.

Next, after drying for 4 hours at 100°C using a convection oven, the dried extrusion-molded catalyst was cut at 2 mm intervals andn calcined for 6 hours at 450°C using a muffle furnace to prepare a magnesium-based extrusion-molded catalyst (A-1).

Comparative Example 1-1. Preparation of Tablet catalyst

Magnesium oxide (MgO powder, Crystallite size 9 nm, BET surface area 144.4 $m^2/g$, Total pore volume 0.72 $cm^3/g$, Pore size 6, 40 nm) was first tableted into a disc shape through a plate-type press machine and an aluminum dish, and this was crushed to prepare granules with an appropriate particle size to impart flowability. 10 wt% of organic binder resin (PPO, polyphenylene oxide) and 1 wt% of lubricant (magnesium stearate) were mixed with the prepared granules, and tablets with a diameter of 2.4 mm were prepared using a single rotary tablet press catalyst molding machine.

Next, after drying the tablet-shaped molded catalyst at 100°C for 4 hours in a convection oven, it was calcined at 450°C for 1 hour in a muffle furnace to obtain the magnesium-based tablet-shaped molded catalyst (B-1).

Examples 1-2 to 1-8

The procedure was carried out in the same manner as in Example 1-1, except that the components and contents of the binder resin and solvent used were changed as shown in Table 1 below.

[TABLE 1]

| Category | Form | binder resin | binder resin |
|---|---|---|---|
| Example 1-1(A-1) | Extrudate | MC 1g | D.I.water 60g |
| Example 1-2(A-2) | Extrudate | MC 1g | D.I.water 60g |
| Example 1-3(A-3) | Extrudate | MC 0.2g | D.I.water 60g |
| Example 1-4(A-4) | Extrudate | MC 2g | D.I.water 60g |
| Example 1-5(A-5) | Extrudate | MC 10g | D.I.water 60g |
| Example 1-6(A-6) | Extrudate | MC 1g | D.I.water 72g |
| Example 1-7(A-7) | Extrudate | PEG 20000 1g | D.I.water 60g |
| Example 1-8(A-8) | Extrudate | PEG 400 1g | D.I.water 60g |
| Comparative Example 1-1(B-1) | Tablet | PPO 10wt%, Mg stearate 1wt% | - |
| <catalyst form><br>Extrudate: extrusion-molded catalyst<br>Tablet: tablet-shaped molded catalyst<br><binder resin><br>MC: methyl cellulose<br>PEG: polyethylene glycol<br>PPO: polyphenylene oxide | | | |

[Experimental Example 1: Analysis of ortho-alkylation reaction catalyst]

(1) BET specific surface area analysis

The catalysts of Example 1 and Comparative Example 1 were subjected to a nitrogen adsorption-desorption analysis experiment to measure the BET specific surface area value according to the BET (Brunauer Emmett Teller) calculation formula from the isothermal adsorption curve and the amount of adsorbed nitrogen at standard temperature and pressure, and the results are shown in Table 2.

The average diameter of the extrudate form of catalyst means the diameter of the circular cross-section of the cylindrical catalyst prepared by the die mounted on the single piston extruder, and the average diameter of the tablet form of catalyst means the diameter of the circular cross-section of the cylindrical catalyst prepared by the punch mounted on the rotary tablet press.

[TABLE 2]

| Category | Size(average diameter)/Form | BET specific surface area($m^2/g$) |
|---|---|---|
| Example 1-1(A-1) | 2 mm/Extrudate | 77.86 |
| Example 1-2(A-2) | 1.2 mm/Extrudate | 60.32 |

(continued)

| Category | Size(average diameter)/Form | BET specific surface area($m^2$/g) |
|---|---|---|
| Example 1-3(A-3) | 2 mm/Extrudate | 129.82 |
| Example 1-4(A-4) | 2 mm/Extrudate | 129.42 |
| Example 1-5(A-5) | 2 mm/Extrudate | 71.87 |
| Example 1-6(A-6) | 2 mm/Extrudate | 131.17 |
| Example 1-7(A-7) | 2 mm/Extrudate | 45.09 |
| Example 1-8(A-8) | 2 mm/Extrudate | 83.71 |
| Comparative Example 1-1(B-1) | 2.4 mm/Tablet | 82.40 |

(2) Hg porosity analysis

The pore structures in the macropores and mesopore regions of the catalysts of Example 1 and Comparative Example 1 were confirmed through Mercury porosimetry, and the results are shown in Table 3 and FIG. 3.

[TABLE 3]

| Category | macropores (mL/g)/(vol%)* | mesopores (mL/g)/(vol%)* | Bulk density (mL/g) | Apparent density (g/mL) |
|---|---|---|---|---|
| Example 1-1(A-1) | 0.0153/2.63 | 0.5662/97.37 | 1.0082 | 3.0764 |
| Example 1-2(A-2) | 0.0153/2.76 | 0.5389/97.24 | 0.8948 | 2.6645 |
| Example 1-3(A-3) | 0.0187/3.04 | 0.5974/96.96 | 0.9159 | 2.7116 |
| Example 1-5(A-5) | 0.0558/8.84 | 0.5753/91.16 | 0.9671 | 3.6060 |
| Example 1-6(A-6) | 0.0575/7.17 | 0.7441/92.83 | 0.7807 | 2.8106 |
| Comparative Example 1-1(B-1) | 0.0799/17.04 | 0.3889/82.96 | 1.1214 | 3.0060 |
| * Means the ratio with respect to the total pore volume of the macropores and the mesopores | | | | |

As can be seen from Tables 2 and 3 and Figure 3, to minimize loss of activity due to internal and external mass diffusion resistance, it is preferable to prepare an extrusion-molded catalyst in the form of an extrudate using an appropriate organic binder resin and solvent as defined herein. Accordingly, it was confirmed that appropriate pores were formed in the mesopore-to-micropore range, and that the BET specific surface area was within an appropriate range.

By achieving the corresponding pore distribution and BET specific surface area value, the mass diffusion resistance during the alkylation reaction can be minimized, leading to a high conversion rate and selectivity. This will be described in more detail in Experimental Example 2.

[Example 2 and Comparative Example 2]

Example 2-1: Preparation of alkylation reaction product of m-cresol

The alkylation reaction of m-cresol was carried out through a continuous flow gas-phase reaction apparatus. First, 1 g of the molded catalyst from Example 1-1 having the physical properties of Table 1 was packed into a stainless steel tube type reactor of 1/2 inch diameter x 50 cm length, and then installed inside a main furnace.

Thereafter, $N_2$, the carrier gas, was flowed using a mass flow controller (MFC) (37.5 cc/min), and the inside of the reactor was raised to the activation temperature (450°C) and maintained for 1 hour for catalyst activation. Subsequently, a monomer composition (12.5 mg/min, m-cresol : Methanol : D.I.water = 1:5:1, LHSV 0.75/h) was passed through a pre-heater (250°C), vaporized, and then injected. A reaction composition comprising the catalyst and the monomer composition was injected into the reactor, and then, an alkylation reaction (450°C, maintained at atmospheric pressure) was carried out for 5 hours to obtain a reaction product.

The product was collected in a liquid form (using IPA (isopropyl alcohol) as a solvent) during the reaction for analysis.

Examples 2-2 to 2-8, Comparative Example 2-1

The alkylation reaction was carried out in the same manner as in Example 2-1, except that the catalyst was changed from the catalyst of Example 1-1 to the catalysts of Examples 1-2 to 1-8 and Comparative Example 1-1, from Table 1, to obtain the reaction product.

[Experimental Example 2: Analysis of ortho-alkylation reaction products]

(2-1) Analysis of reaction products by catalyst type

The liquid containing the reaction products prepared in Example 2 and Comparative Example 2 was analyzed by gas chromatography (GC) using a gas chromatograph equipped with a flame ionization detector (FID). From the GC data, the m-cresol conversion rate and the selectivity for the target products (2,3,6-TMP, 2,5-DMP, 2,3-DMP, tetramethylphenol) were calculated using Equations 1 and 2 below, and the results are shown in Table 4, FIG. 1 (catalyst type (size/shape)), and FIG. 2 (type/content of binder resin used in catalyst preparation).

The Target product was defined as the final target product 2,3,6-TMP and the intermediate substances 2,5-DMP and 2,3-DMP, from which the final product can be obtained through additional reaction.

[Equation 1]

$$\text{Conversion rate of m-cresol(\%)} = \frac{\text{Moles of reacted m-cresol}}{\text{Moles of supplied m-cresol}} \times 100$$

[Equation 2]

$$\text{Target product selectivity (\%)} = \frac{\text{Moles of 2,5-DMP + 2,3-DMP + 2,3,6-TMP produced}}{\text{Moles of reacted m-cresol}} \text{X } 100$$

[TABLE 4]

| Category(catalyst type) | Conversi on rate | Selectivity | | | |
|---|---|---|---|---|---|
| | m-cresol | Target product[a] | DMP[b] | TMP[c] | TeMP[d] |
| Example 2-1(A-1) | 96.13 | 88.51 | 21.15 | 62.45 | 8.59 |
| Example 2-2(A-2) | 93.58 | 95.39 | 37.91 | 48.87 | 2.2 |
| Example 2-3(A-3) | 97.91 | 85.25 | 15.71 | 66.02 | 11.57 |
| Example 2-4(A-4) | 97.14 | 87.13 | 19.21 | 63.65 | 9.77 |
| Example 2-5(A-5) | 93.50 | 89.94 | 27.2 | 56.57 | 6.98 |
| Example 2-6(A-6) | 99.40 | 83.58 | 9.98 | 71.51 | 13.52 |
| Example 2-7(A-7) | 93.74 | 91.96 | 29.14 | 56.10 | 5.72 |
| Example 2-8(A-8) | 94.95 | 93.99 | 26.47 | 61.76 | 4.69 |
| Comparative Example 2-1(B-1) | 85.01 | 92.68 | 29.16 | 56.0 | 4.82 |
| [a] Target product : DMP and TMP<br>[b] DMP : 2,5-dimethylphenol and 2,3-dimethylphenol<br>c TMP : 2,3,6-trimethylphenol<br>d TeMP : Tetra- methyl phenol | | | | | |

As can be confirmed from the experimental data in Table 4, when using the extrusion-molded catalyst according to the present invention, high catalytic activity was exhibited even with a single catalyst, and when used to prepare an ortho-alkylation reaction product, it was confirmed that remarkably high selectivity and conversion rate can be exhibited.

In the case of Comparative Example 2-1, by using a tablet-shaped molded catalyst, it was difficult to achieve the desired level of catalytic activity, and accordingly, it was confirmed that the conversion rate, etc., was lower compared to the Examples.

(2-2) Analysis of product(s) by reaction conditions

Example 3: Changing reaction temperature

An alkylation reaction was performed in the same manner as in Example 2-1, except that the methylation reaction temperature was started at 400°C and increased by 10°C every 3 hours up to 450°C, and according to the method of Experimental Example 2 described above, the m-cresol conversion rate and the selectivity of the Target product (2,3,6-TMP, 2,5-DMP, 2,3-DMP) were calculated, and the results are shown in Table 5 below.

Example 4: Changing carrier gas flow rate

An alkylation reaction was performed in the same manner as in Example 2-1, except that the carrier gas flow rate was 18.7 cc/min, and according to the method of Experimental Example 2 described above, the m-cresol conversion rate and the selectivity of the Target product (2,3,6-TMP, 2,5-DMP, 2,3-DMP) were calculated, and the results are shown in Table 5 below.

Example 5: Changing the amount of Methanol injection

An alkylation reaction was performed in the same manner as in Example 1, except that the liquid mixture comprising the reactant was injected with m-cresol : Methanol : D.I.water = 1:4:1 (11.1 mg/min, LHSV 0.666/h) and m-cresol : Methanol : D.I.water = 1:3:1 (9.7 mg/min, LHSV 0.582/h), and according to the method of Experimental Example 2 described above, the m-cresol conversion rate and the selectivity of the Target product (2,3,6-TMP, 2,5-DMP, 2,3-DMP) were calculated, and the results are shown in Table 5 below.

[TABLE 5]

| Category (catalyst type) | Reaction condition | Conversi on rate | Selectivity | | | |
|---|---|---|---|---|---|---|
| | | m-cresol | Target product[a] | DMP[b] | TMP[c] | TeMP[d] |
| Example 3 (A-1) Reac-tion tem-perature control | 400 °C | 47.12 | 97.16 | 82.32 | 14.84 | 0 |
| | 410 °C | 60.58 | 97.22 | 76.26 | 20.96 | 0.49 |
| | 420 °C | 70.09 | 96.97 | 67.5 | 29.47 | 0.98 |
| | 430 °C | 84.91 | 95.28 | 46.16 | 49.12 | 2.93 |
| | 440 °C | 90.38 | 94.12 | 36.22 | 57.9 | 4.06 |
| | 450 °C | 95.55 | 92.41 | 24.74 | 67.67 | 5.75 |
| Example 4 (A-1) Nitrogen flow | 37.5 cc/min (same as Example 2-1) | 96.13 | 88.51 | 21.15 | 62.45 | 8.59 |
| rate control | 18.7 cc/min | 98.76 | 87.67 | 12.65 | 75.02 | 9.93 |
| Example 5 (A-1) Feed ratio control | 1:3:1 | 59.98 | 96.14 | 75.45 | 20.69 | 0.56 |
| | 1:4:1 | 96.29 | 89.61 | 26.95 | 62.66 | 7.54 |
| | 1:5:1(same as Example 2-1) | 96.13 | 88.51 | 26.06 | 62.45 | 8.59 |

[a] Target product : DMP and TMP
[b] DMP : 2,5-dimethylphenol and 2,3-dimethylphenol
c TMP : 2,3,6-trimethylphenol
d TeMP : Tetra- methyl phenol

As can be confirmed from the experimental data in Table 5, in the case of Example 3, the methylation reaction temperature was started at 400°C and increased by 10°C every 3 hours up to 450°C, and through this, it was confirmed that the catalyst performance increases with the reaction temperature.

In Example 4, the flow rate of the nitrogen feed was changed, and through this the effect on the contact time between the catalyst and the reactants (GHSV, gas hourly space velocity) could be confirmed

In the case of Example 5, the ratio of the injected Feed was changed, and through this, it was confirmed that there is an influence of the ratio of the reactant m-cresol and the alkylating agent methanol.

## Claims

1. An extrusion-molded catalyst for ortho-alkylation reaction,

    comprising magnesium oxide;
    comprising macroporess having a diameter of 50 nm to 10,000 nm and mesopores having a diameter of 2 nm to 50 nm; and
    having a BET specific surface area of 45 $m^2/g$ to 180 $m^2/g$.

2. The extrusion-molded catalyst for ortho-alkylation reaction according to Claim 1,

    wherein with respect to the total pore volume of the macropores and the mesopores,
    the macroporess are comprised in an amount of 1 to 10 vol%, and
    the mesopores are comprised in an amount of 90 to 99 vol%.

3. The extrusion-molded catalyst for ortho-alkylation reaction according to Claim 1, wherein the size of the extrusion-molded catalyst is 0.5 mm to 6.0 mm.

4. A preparation method of the extrusion-molded catalyst for ortho-alkylation reaction according to claim 1, comprising:

    a first step of preparing a mixture of magnesium oxide having a bimodal pore structure and a BET specific surface area of 100 $m^2/g$ to 180 $m^2/g$, an organic binder resin, and a solvent; and
    a second step of extrusion molding the mixture.

5. The preparation method of the extrusion-molded catalyst for ortho-alkylation reaction according to Claim 4, wherein the bimodal pore structure of the magnesium oxide comprises a first pore having a diameter of 2 nm to 10 nm, and a second pore having a diameter of 10 nm to 50 nm.

6. The preparation method of the extrusion-molded catalyst for ortho-alkylation reaction according to Claim 4,

    based on 100 parts by weight of magnesium oxide,
    the organic binder resin is included in the amount of 0.1 to 20 parts by weight, and
    the solvent is included in the amount of 50 to 200 parts by weight.

7. The preparation method of the extrusion-molded catalyst for ortho-alkylation reaction according to Claim 4, wherein the organic binder resin comprises one or more selected from the group consisting of methyl cellulose, carboxymethyl cellulose, ethylene glycol, polyethylene glycol, polyphenylene oxide, glycerin, and propylene glycol.

8. The preparation method of the extrusion-molded catalyst for ortho-alkylation reaction according to Claim 4, wherein the solvent is alcohol, water, or a mixture thereof.

9. The preparation method of the extrusion-molded catalyst for ortho-alkylation reaction according to Claim 4, wherein the solvent is water.

10. The preparation method of the extrusion-molded catalyst for ortho-alkylation reaction according to Claim 4, wherein the extrusion molding of the second step is performed using a single piston extrusion molding machine.

11. The preparation method of the extrusion-molded catalyst for ortho-alkylation reaction according to Claim 4, further comprising a step of drying and calcination after the extrusion molding of the second step.

12. The preparation method of the extrusion-molded catalyst for ortho-alkylation reaction according to Claim 11, wherein the drying is performed at 80°C to 120°C.

13. The preparation method of the extrusion-molded catalyst for ortho-alkylation reaction according to Claim 11, wherein the calcination is performed at 300°C to 600°C.

14. An ortho-alkylation reaction composition, comprising
the extrusion-molded catalyst for ortho-alkylation reaction according to Claim 1 and a monomer composition comprising a meta-alkyl substituted phenolic monomer.

15. A preparation method of an ortho-alkylation reaction product, comprising
in the presence of the extrusion-molded catalyst for ortho-alkylation reaction according to Claim 1, an alkylation reaction of a monomer composition comprising a meta-alkyl substituted phenolic monomer.

【FIG. 1】

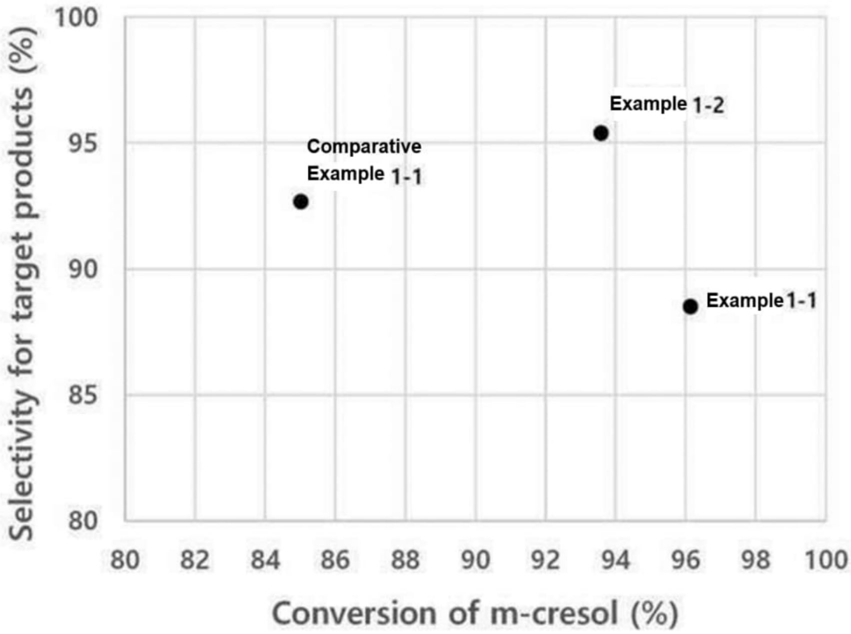

Selectivity for target products (%)
100
95
90
85
80
80 82 84 86 88 90 92 94 96 98 100
Conversion of m-cresol (%)
Comparative Example 1-1
Example 1-2
Example 1-1


【FIG. 2】

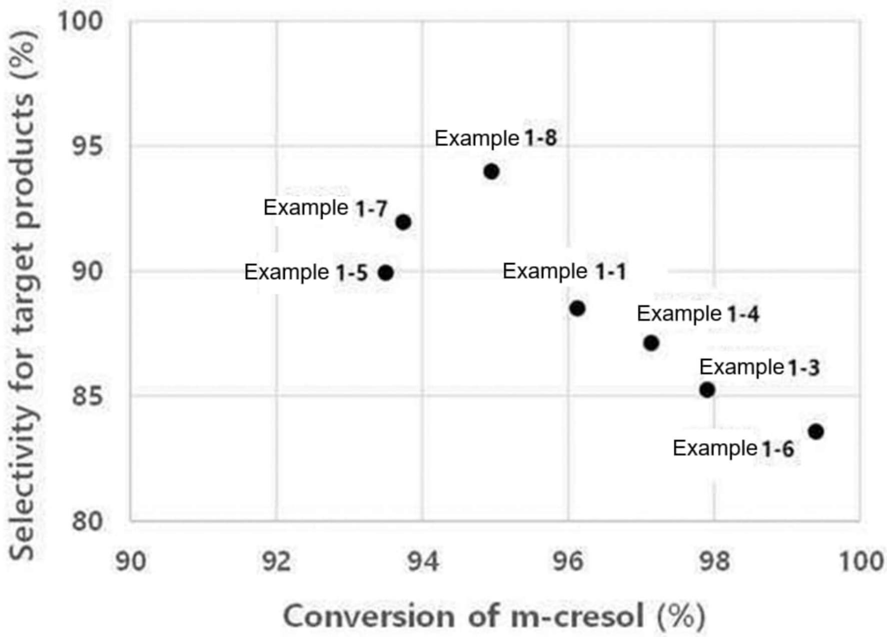

Selectivity for target products (%)
100
95
90
85
80
90 92 94 96 98 100
Conversion of m-cresol (%)
Example 1-8
Example 1-7
Example 1-5
Example 1-1
Example 1-4
Example 1-3
Example 1-6

【FIG. 3】

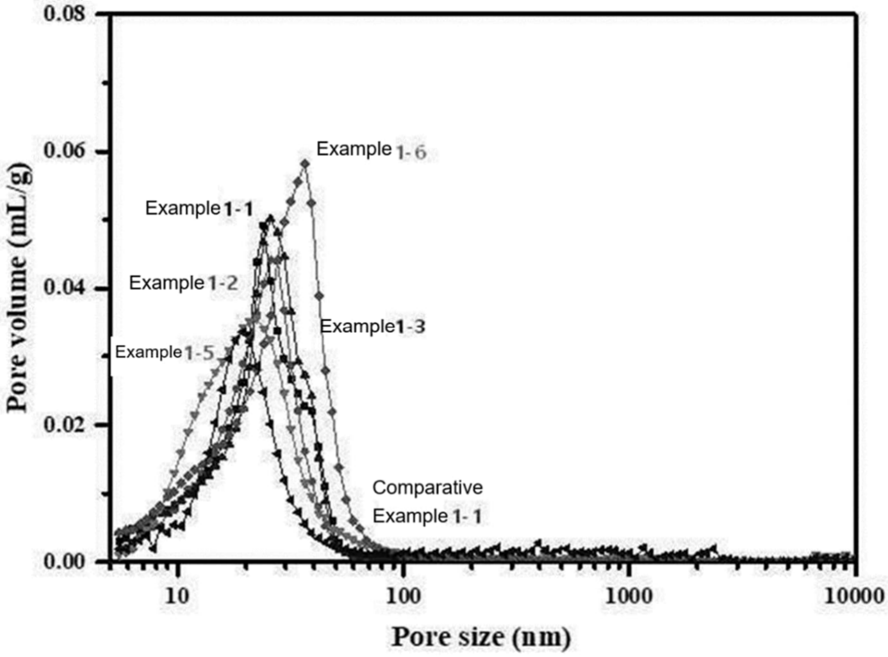
0.08
0.06
0.04
0.02
0.00
Pore volume (mL/g)
10
100
1000
10000
Pore size (nm)
Example 1-6
Example 1-1
Example 1-2
Example 1-3
Example 1-5
Comparative
Example 1-1

## INTERNATIONAL SEARCH REPORT

International application No. **PCT/KR2024/004910**

**A. CLASSIFICATION OF SUBJECT MATTER**

**B01J 23/02**(2006.01)i; **B01J 35/64**(2024.01)i; **B01J 35/61**(2024.01)i; **B01J 37/00**(2006.01)i; **C07C 37/11**(2006.01)i; **C07C 39/06**(2006.01)i

According to International Patent Classification (IPC) or to both national classification and IPC

**B. FIELDS SEARCHED**

Minimum documentation searched (classification system followed by classification symbols)

B01J 23/02(2006.01); B01J 19/12(2006.01); B01J 21/10(2006.01); B01J 23/755(2006.01); B01J 23/78(2006.01); B01J 35/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 산화 마그네슘(magnesium oxide, MgO), 마크로 기공(macro pore), 메조 기공(meso pore), 직경(diameter), 비표면적(specific surface area), 촉매(catalyst), 압출(extrusion), 오르토-알킬화(ortho-alkylation)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2013-0126437 A (KOREA INSTITUTE OF SCIENCE AND TECHNOLOGY) 20 November 2013 (2013-11-20)<br>See paragraphs [0038]-[0068]; example 2; and table 2. | 1-15 |
| Y | US 6261987 B1 (WATSON, B. A. et al.) 17 July 2001 (2001-07-17)<br>See abstract; and column 6, lines 40-43. | 1-15 |
| Y | KR 10-2004-0045497 A (GENERAL ELECTRIC COMPANY) 01 June 2004 (2004-06-01)<br>See paragraphs [0001] and [0029]; and claims 19-21. | 5 |
| A | US 6951966 B1 (HIGASHI, M. et al.) 04 October 2005 (2005-10-04)<br>See entire document. | 1-15 |
| A | KR 10-2013-0066916 A (KOREA INSTITUTE OF ENERGY RESEARCH) 21 June 2013 (2013-06-21)<br>See entire document. | 1-15 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 July 2024** | **18 July 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/004910**

| Patent document cited in search report | Publication date (day/month/year) | Patent family member(s) | Publication date (day/month/year) |
|---|---|---|---|
| KR 10-2013-0126437 A | 20 November 2013 | KR 10-1466125 B1 | 27 November 2014 |
| | | US 2013-0303357 A1 | 14 November 2013 |
| | | US 9180437 B2 | 10 November 2015 |
| US 6261987 B1 | 17 July 2001 | AU 2926001 A | 12 September 2001 |
| | | CN 1213801 C | 10 August 2005 |
| | | CN 1406152 A | 26 March 2003 |
| | | EP 1263529 A1 | 11 December 2002 |
| | | EP 1263529 B1 | 18 November 2009 |
| | | JP 2004-500238 A | 08 January 2004 |
| | | KR 10-0720218 B1 | 21 May 2007 |
| | | KR 10-2002-0080443 A | 23 October 2002 |
| | | US 2002-0128431 A1 | 12 September 2002 |
| | | US 2002-0128432 A1 | 12 September 2002 |
| | | US 6294499 B1 | 25 September 2001 |
| | | US 6395871 B1 | 28 May 2002 |
| | | US 6492300 B2 | 10 December 2002 |
| | | US 6620908 B2 | 16 September 2003 |
| | | WO 01-64334 A1 | 07 September 2001 |
| KR 10-2004-0045497 A | 01 June 2004 | CN 1633335 A | 29 June 2005 |
| | | CN 1633335 B | 16 June 2010 |
| | | EP 1438131 A1 | 21 July 2004 |
| | | JP 2005-505407 A | 24 February 2005 |
| | | US 2003-0073572 A1 | 17 April 2003 |
| | | US 2005-0004407 A1 | 06 January 2005 |
| | | US 6897175 B2 | 24 May 2005 |
| | | US 7208438 B2 | 24 April 2007 |
| | | WO 03-031057 A1 | 17 April 2003 |
| US 6951966 B1 | 04 October 2005 | JP 2000-167396 A | 20 June 2000 |
| | | JP 3995849 B2 | 24 October 2007 |
| KR 10-2013-0066916 A | 21 June 2013 | KR 10-1394287 B1 | 13 May 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230055405